# EUROPEAN PATENT APPLICATION

(11) **EP 4 389 181 A1**
(43) Date of publication of application: **26.06.2024**
(21) Application number: 21953768.5
(22) Date of filing: 19.08.2021
(51) Int. Cl.: A61M 16/00

(54) **MEDICAL VENTILATION APPARATUS**

(71) Applicant: Shenzhen Mindray Bio-Medical Electronics Co., Ltd, Shenzhen, Guangdong 518057 (CN)
(72) Inventor: HUANG, Jiping, Shenzhen, Guangdong 518057 (CN); PAN, Ruiling, Shenzhen, Guangdong 518057 (CN); TIAN, Wentao, Shenzhen, Guangdong 518057 (CN)
(74) Representative: KIPA AB
(86) International application number: PCT/CN2021/113602
(87) International publication number: WO 2023/019525

(57) **Abstract**

A medical ventilation apparatus (100, 200, 300). The medical ventilation apparatus (100, 200, 300) is used for being communicationally connected to a monitor; the medical ventilation apparatus (100, 200, 300) is configured to perform respiratory therapy on a patient; and the medical ventilation apparatus (100, 200, 300) comprises a display device for displaying respiratory therapy parameters of the medical ventilation apparatus (100, 200, 300). The medical ventilation apparatus (100, 200, 300) is further used for obtaining a control instruction inputted by a user, and at least controlling, on the basis of the control instruction, the monitor to work. The medical ventilation apparatus (100, 200, 300) can control the monitor to improve the operation efficiency in the process of ventilation and monitoring and reduce the workload of medical staff.

## Description

### TECHNICAL FIELD

The disclosure relates to a technical field of ventilation treatment, and more particularly to a medical ventilation apparatus.

### BACKGROUND

For patients using mechanical ventilation in clinical practice, it is usually necessary to monitor their vital signs by monitors. The purpose of mechanical ventilation is to maintain oxygenation of patient and keep vital signs of patient in a stable state, so that a doctor usually adjusts a ventilation treatment strategy for the patient based on information monitored by the monitor. Therefore, the uses of monitor and ventilator are closely related and inseparable.

Currently, ventilators and monitors are two completely independent apparatuses. Usually, each mechanically ventilated patient is equipped with one monitor and one ventilator at his/her bedside. The two apparatuses are prepared to be started, set up, installed, and fixed separately, such cumbersome operations and complicated apparatus management increase the workload of medical staff. Therefore, the ventilation apparatus needs to be improved to solve the current problems.

### SUMMARY

A series of simplified concepts are introduced into summary of this disclosure, and are further elaborated in specific embodiments of this disclosure. The summary of this disclosure does not attempt to define key and necessary technical characteristics of the claimed technical solution, nor attempt to determine a protection scope of the claimed technical solution.

A first aspect according to an embodiment of this disclosure provides a medical ventilation apparatus, including: a patient pipeline, which is connected with a patient interface, so as to deliver a ventilation gas to an airway of a patient; a pressure generator, which is configured to generate the ventilation gas with a preset pressure and deliver said ventilation gas to the patient; a controller which is configured to control the pressure generator to ventilate the patient based on configuration information which matches with the patient; a human-machine interaction interface, which is configured to display a respiratory treatment parameter of the medical ventilation apparatus and to acquire input information from a user; wherein the medical ventilation apparatus is further provided with a physical connection structure, which is configured to assemble a monitor with the medical ventilation apparatus, which assembly is capable of being disassembled; wherein the monitor is configured to measure a physiological parameter of the patient, when the medical ventilation apparatus implements a ventilation treatment on the patient; wherein the medical ventilation apparatus further includes a communication interface, which is configured to implement information communication with the monitor, when the monitor is assembled with the medical ventilation apparatus.

A second aspect according to an embodiment of this disclosure provides a medical ventilation apparatus, including: a patient pipeline, which is connected with a patient interface, so as to deliver a ventilation gas to an airway of a patient; a pressure generator, which is configured to generate the ventilation gas with a preset pressure and deliver said ventilation gas to the patient; a controller which is configured to control the pressure generator to ventilate the patient based on configuration information which matches with the patient; a human-machine interaction interface, which is configured to display a respiratory treatment parameter of the medical ventilation apparatus and to acquire input information from a user; wherein the medical ventilation apparatus further includes a communication interface, which is configured to implement information communication with a monitor; wherein the monitor is configured to measure a physiological parameter of the patient, when the medical ventilation apparatus implements a ventilation treatment on the patient; the human-machine interaction interface is further configured to acquire a control instruction which is inputted by the user, and the controller is further configured to transmit the control instruction to the monitor through the communication interface according to the control instruction, so as to control the monitor to perform a first operation.

A third aspect according to an embodiment of this disclosure provides a medical ventilation apparatus, including: a patient pipeline, which is connected with a patient interface, so as to deliver a ventilation gas to an airway of a patient; a pressure generator, which is configured to generate the ventilation gas with a preset pressure and deliver said ventilation gas to the patient; a controller which is configured to control the pressure generator to ventilate the patient based on configuration information which matches with the patient; a human-machine interaction interface, which is configured to display a respiratory treatment parameter of the medical ventilation apparatus and to acquire input information from a user; wherein the medical ventilation apparatus further includes a communication interface, which is configured to implement information communication with a monitor; wherein the monitor is configured to measure a physiological parameter of the patient, when the medical ventilation apparatus implements a ventilation treatment on the patient; the controller is further configured to acquire, through the communication interface, the physiological parameter of the patient, which parameter is measured by the monitor; the human-machine interaction interface is further configured to display the acquired physiological parameter.

A fourth aspect according to an embodiment of this disclosure provides a medical device, including: a ventilation apparatus and a monitoring apparatus; wherein the monitoring apparatus is integrated with the ventilation apparatus where the monitoring apparatus is not capable of being dissembled from the ventilation apparatus; wherein the ventilation apparatus includes a patient pipeline, which is connected with a patient interface, so as to deliver a ventilation gas to an airway of a patient; a pressure generator, which is configured to generate the ventilation gas with a preset pressure and deliver said ventilation gas to the patient; wherein the monitoring apparatus is configured to acquire a physiological parameter of the patient through a physiological parameter sensor; wherein the medical device further includes a controller which is configured to control the ventilation apparatus to ventilate the patient based on configuration information which matches with the patient, and configured to control the monitoring apparatus to acquire the physiological parameter of the patient; and a human-machine interaction interface, which includes a first display screen and a second display screen, wherein the first display screen is configured to display a respiratory treatment parameter of the ventilation apparatus, the second display screen is configured to display the physiological parameter.

A fifth aspect according to an embodiment of this disclosure provides a medical ventilation apparatus, which is in communication connection with a monitor and is configured to implement a respiratory treatment on a patient; wherein the medical ventilation apparatus includes a display apparatus, which is configured to display a respiratory treatment parameter of the medical ventilation apparatus; wherein the medical ventilation apparatus is further configured to acquire a control instruction which is inputted by a user, so as to at least control the monitor to work based on the control instruction.

A sixth aspect according to an embodiment of this disclosure provides a medical ventilation apparatus, which is in communication connection with a monitor and is configured to implement a respiratory treatment on a patient; wherein the medical ventilation apparatus includes a display apparatus, which is configured to display a respiratory treatment parameter of the medical ventilation apparatus; wherein the medical ventilation apparatus is further configured to acquire a physiological parameter of the patient, which parameter is monitored by the monitor, and to display the physiological parameter through the display apparatus.

A seventh aspect according to an embodiment of this disclosure provides a medical ventilation apparatus, which is in communication connection with a monitor and is configured to implement a respiratory treatment on a patient; wherein the monitor is configured to monitor a physiological parameter of the patient; the medical ventilation apparatus is further configured to acquire a control instruction which is inputted by a user, and to control the monitor to enter into a CPR rescue mode based on the control instruction.

In the embodiment of this disclosure, the medical ventilation apparatus is in communication connection with the monitor, and is configured to acquire a control instruction which is inputted by a user and at least control the monitor to work based on the control instruction, such that the medical ventilation apparatus can control the monitor to improve operation efficiency during ventilation and monitoring and reduce the workload of medical staff.

### BRIEF DESCRIPTION OF THE DRAWINGS

In order to provide a clearer explanation of technical solutions in embodiments of this disclosure, a brief introduction is given to the accompanying drawings required in the description of the embodiments. It is evident that the accompanying drawings in the following description are only some embodiments of this disclosure. For ordinary technical personnel in the art, other accompanying drawings can be acquired based on these drawings without any creative effort.
FIG. 1 is a block diagram of a medical ventilation apparatus according to an embodiment of this disclosure.
FIG. 2 is a block diagram of a medical ventilation apparatus according to an embodiment of this disclosure.
FIG. 3 is a block diagram of a medical ventilation apparatus according to an embodiment of this disclosure.
FIG. 4 is a block diagram of a medical device according to an embodiment of this disclosure.
FIG. 5 is a structural diagram of a medical ventilation apparatus according to an embodiment of this disclosure.
FIG. 6 is a page display diagram of a display apparatus of a medical ventilation apparatus according to an embodiment of this disclosure.
FIG. 7 is a page display diagram of a display apparatus of a medical ventilation apparatus according to another embodiment of this disclosure.
FIG. 8 is a page display diagram of a display apparatus of a medical ventilation apparatus according to a further embodiment of this disclosure.

### DESCRIPTION OF THE PREFERRED EMBODIMENTS

In order to make the purpose, technical solution, and advantages of this disclosure more obvious, the following refers to the attached drawings to describe in detail the exemplary embodiments according to this disclosure. Obviously, the described embodiments are only some of the embodiments of this disclosure, not all of them. It should be understood that this disclosure is not limited by the embodiments described here. Based on the embodiments described in this disclosure, all other embodiments acquired by those skilled in the art without creative work, fall within the protection scope of this disclosure.

In the following description, a large number of specific details are provided to provide a more thorough understanding of this disclosure. However, it is evident to those skilled in the art that this disclosure can be implemented without the need for one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

The purpose of the terminology used here is only to describe specific embodiments and is not a limitation of this disclosure. When used here, the singular forms of "an", "a", and "the/said" are also intended to include the plural form, unless the context clearly indicates another way. It should also be understood that the terms, such as "including", "and/or", "comprising", when used in this disclosure, determine the presence of the characteristics, integers, steps, operations, components, and/or elements, but do not exclude the presence or addition of one or more other characteristics, integers, steps, operations, components, and/or elements. When used here, the term "and/or" includes any and all combinations of related listed items.

In order to fully understand this disclosure, a detailed structure is provided in the following description to explain the technical solution proposed in this disclosure. The optional embodiments of this disclosure are described in detail below, however, in addition to these detailed description, this disclosure may also have other embodiments.

The current ventilation apparatus and monitors are set up separately, so there are the following problems. The two apparatuses need to be prepared and started separately, which increases operation steps of medical staff and is cumbersome and time-consuming.

As the two apparatuses are set up separately, some settings of the two apparatuses for the same patient are repeated, such as patient information settings and device interconnection settings (connection to a central station, connection to information system), which increases the workload of medical staff.

In addition, a bedside space is limited, and the installation and fixation of the two apparatuses not only take up space but also increase the installation and fixation operations of medical staff.

In addition, in some scenarios, an adjustment of ventilator treatment strategy is based on vital signs of patient, which are monitored by the monitor, so it is necessary to frequently switch the line of sight when operating the ventilator. For example, in pre-hospital first aid or transfer scenarios, since the two apparatuses are set up separately, two apparatuses need to be carried, which increases the intensity of apparatus management. Moreover, the apparatuses are also relatively bulky.

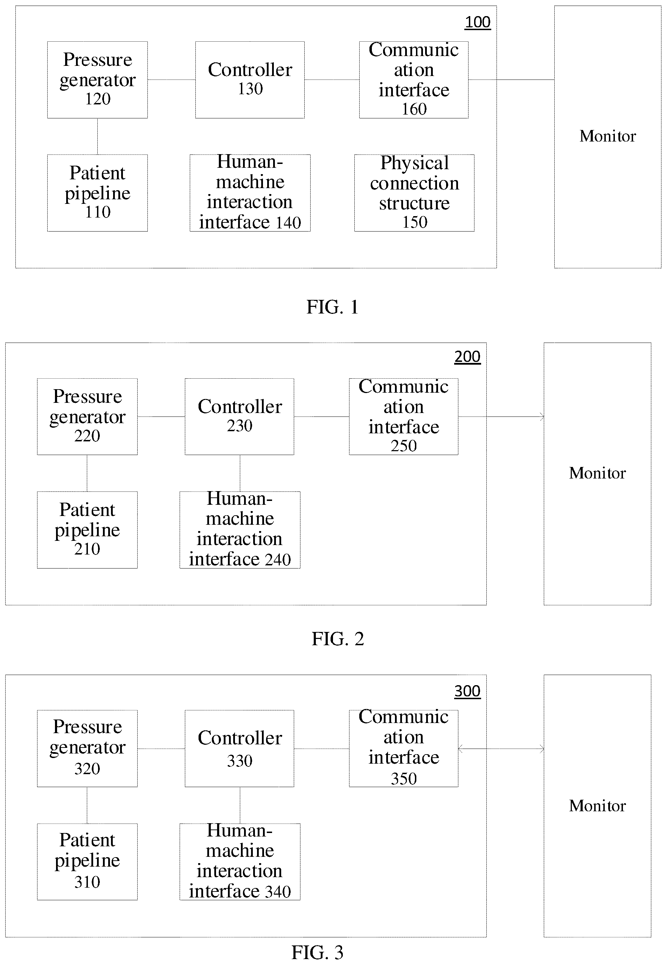

In order to solve these technical problems, a first aspect according to an embodiment of this disclosure provides a medical ventilation apparatus 100. Referring to FIG. 1, the medical ventilation apparatus includes a patient pipeline 110 which is connected with a patient interface, so as to deliver a ventilation gas to an airway of a patient; a pressure generator 120 which is configured to generate the ventilation gas at a preset pressure and deliver said ventilation gas to the patient; a controller 130 which is configured to control the pressure generator to ventilate the patient based on configuration information which matches with the patient; a human-machine interaction interface 140 which is configured to display a respiratory treatment parameter of the medical ventilation apparatus 100 and acquire input information from a user. Wherein the medical ventilation apparatus 100 is further provided with a physical connection structure 150 which is configured to assemble a monitor with the medical ventilation apparatus, which assembly is capable of being disassembled. Wherein the monitor is configured to measure a physiological parameter of the patient, when the medical ventilation apparatus implements a ventilation treatment on the patient. Wherein the medical ventilation apparatus further includes a communication interface 160, which is configured to implement information communication with the monitor, when the monitor is assembled with the medical ventilation apparatus 100.

In this embodiment, the monitor is assembled with the medical ventilation apparatus through the physical connection structure 150, so that in a scenario where the medical ventilation apparatus and the monitor need to be used simultaneously, the two apparatuses are equivalent to one apparatus. Different from the way two apparatuses are simply connected through cables, it alleviates the problem of managing two independent apparatuses.

In one embodiment, the human-machine interaction interface 140 is further configured to acquire a control instruction which is inputted by the user, and the controller 130 is further configured to transmit the control instruction to the monitor through the communication interface 160, so as to control the monitor to perform a first operation. Accordingly, the user can operate the monitor directly through the medical ventilation apparatus without the need to frequently switch the apparatuses. Exemplarily, the first operation includes at least one of turning on, turning off, standing by, entering into a designated work mode, and setting an alarm limit.

In one embodiment, the controller 130 is further configured to control the medical ventilation apparatus to perform a second operation according to the received control instruction, and the first operation is correlated with the second operation. Accordingly, the user only needs to input the control instruction once to control both the medical ventilation apparatus and the monitor simultaneously, which improves the operation efficiency of the user during the monitoring and ventilation process. Exemplarily, the second operation includes at least one of turning on, turning off, standing by, entering into a designated work mode, and setting an alarm limit.

In one embodiment, the human-machine interaction interface 140 includes a work mode key, which is correlated with a corresponding designated work mode of the monitor and of the medical ventilation apparatus 100. The work mode key is configured to generate, based on a user operation, a control instruction which is configured to simultaneously control the monitor and the medical ventilation apparatus to enter into said designated work mode.

For example, the work mode key may include a rescue mode key, which is correlated with a cardiopulmonary resuscitation rescue mode of the monitor and a cardiopulmonary resuscitation ventilation mode of the medical ventilation apparatus. In a cardiopulmonary resuscitation (CPR) scenario, when determining that the user operates the rescue mode key, the monitor starts the cardiopulmonary resuscitation rescue mode, and the medical ventilation apparatus simultaneously starts the cardiopulmonary resuscitation ventilation mode. When the monitor starts the cardiopulmonary resuscitation rescue mode, for example, it can start a rescue assistant unit to record a rescue process, and start an effect evaluation unit to evaluate an effect of a cardiopulmonary resuscitation rescue. The cardiopulmonary resuscitation rescue mode of the monitor can record a CPR rescue process and evaluate the effect of CPR rescue through CQI (CPR quality index). Accordingly, the user only needs to operate the rescue mode key to simultaneously start the cardiopulmonary resuscitation rescue mode of the monitor and start the cardiopulmonary resuscitation ventilation mode of the medical ventilation apparatus, which reduces the number of steps to operate the apparatuses during the CPR rescue process and allows medical staff to have more focus on patient.

For example, the controller 130 is further configured to acquire a physiological parameter which is acquired by monitoring the patient with the monitor, and the human-machine interaction interface 140 is further configured to display the acquired physiological parameter. Physiological parameter includes but is not limited to CO₂, ECG, pulse, etc. The human-machine interaction interface 140 is configured to display real-time waveform(s) and real-time value(s) of the physiological parameter, and to mark corresponding safety limit(s) in the real-time waveform(s) simultaneously. For example, the monitor may be a multi-parameter monitor, which can acquire multiple physiological parameters of the patient and monitor the multiple physiological parameters. Exemplarily, the multiple physiological parameters acquired by the multi-parameter monitor at least include an ECG parameter. Medical ventilation apparatus 100 may be a portable ventilator.

Exemplarily, the human-machine interaction interface 140 includes a display apparatus which may include a touch display screen. The display apparatus includes at least two display areas, wherein the physiological parameter and the respiratory treatment parameter are displayed in different areas. Displaying the physiological parameter and the respiratory therapy parameter in different areas helps user to quickly distinguish them.

In another embodiment, the display apparatus can also fuse and display the physiological parameter and the respiratory treatment parameter. For example, the display apparatus includes multiple display areas, and different physiological parameters and different respiratory treatment parameters are displayed in different display areas. The medical ventilation apparatus 100 is also configured to fuse and display the correlated physiological parameter and the respiratory treatment parameter in a same display area. In this way, the display areas for different physiological parameters are divided according to the correlation between the parameters, and the correlated physiological parameter and respiratory treatment parameter are displayed in the same display area, which is helpful for the user to quickly view the correlated physiological parameter and respiratory treatment parameter.

In one embodiment, the physical connection structure 150 includes a hot-plug structure. The medical ventilation apparatus 100 is provided with a plug-in slot which supports a hot-plug monitor. The medical ventilation apparatus communicates with the monitor through a communication interface of the plug-in slot. Exemplarily, the communication interface includes at least one of a contact communication interface, an optical communication interface, a Bluetooth communication interface, and a WIFI communication interface. Through the hot-plug interface, the monitor can be plugged into or unplugged from the medical ventilation apparatus without turning off the medical ventilation apparatus, thus never affecting the normal work of the medical ventilation apparatus.

In one embodiment, the medical ventilation apparatus 100 further includes a power supply apparatus and a common power interface. When the monitor is assembled with the medical ventilation apparatus, the power supply apparatus is configured to power the medical ventilation apparatus and the monitor simultaneously, not requiring to separately provide a power supply apparatus and a power interface for the monitor.

In one embodiment, the medical ventilation apparatus includes a portable ventilator and the monitor includes a transport monitor. The transport monitor is configured to monitor patient during transport outside or inside the hospital. The integration of the portable ventilator and the transport monitor helps to reduce the difficulty of apparatus management during the transport process.

In one embodiment, the controller 130 is further configured to transmit work data of the medical ventilation apparatus to the monitor through the communication interface 160, so that the monitor can perform monitoring based on the work data of the medical ventilation apparatus, and the user can also view the work data of the medical ventilation apparatus on the monitor. For example, the work data transmitted to the monitor through the communication interface 160 is at least one of ventilation configuration data and ventilation monitoring data of the medical ventilation apparatus. After the work data of the medical ventilation apparatus is transferred to the monitor, the ventilation configuration data and the ventilation monitoring data can be transferred between medical ventilation apparatuses by unplugging the monitor from one medical ventilation apparatus and then plugging the monitor into another medical ventilation apparatus. The medical ventilation apparatus 100 in the embodiment of this disclosure is physically connected with the monitor through the physical connection structure 150, and is in communication connection with the monitor through the communication interface 160, so that the monitor and the medical ventilation apparatus are physically and communicatively integrated, thereby reducing the volume of the apparatuses, making the apparatuses easy to manage, transport and operate.

A second aspect according to an embodiment of this disclosure provides a medical ventilation apparatus. Referring to FIG. 2, the medical ventilation apparatus 200 includes a patient pipeline 210, which is connected with a patient interface, so as to deliver a ventilation gas to an airway of a patient; a pressure generator 220, which is configured to generate the ventilation gas with a preset pressure and deliver said ventilation gas to the patient; a controller 230, which is configured to control the pressure generator 220 to ventilate the patient based on configuration information which matches with the patient; a human-machine interaction interface 240, which is configured to display a respiratory treatment parameter of the medical ventilation apparatus 200 and to acquire input information from a user. Wherein the medical ventilation apparatus 200 further includes a communication interface 250, which is configured to implement information communication with a monitor. Wherein the monitor is configured to measure a physiological parameter of the patient when the medical ventilation apparatus 200 implements a ventilation treatment on the patient. The human-machine interaction interface 240 is further configured to acquire a control instruction which is inputted by the user, and the controller 230 is further configured to transmit the control instruction to the monitor through the communication interface 250 according to the control instruction, so as to control the monitor to perform a first operation.

In one embodiment, the controller 230 is further configured to control the medical ventilation apparatus 200 to perform a second operation according to the control instruction, and the first operation is correlated with the second operation. Exemplarily, the first operation or the second operation includes at least one of turning on, turning off, standing by, entering into a designated work mode, and setting an alarm limit. Accordingly, the user can control the medical ventilation apparatus and monitor simultaneously by inputting one control instruction, which improves operation efficiency of apparatuses.

Exemplarily, the human-machine interaction interface 240 includes a work mode key, which is correlated with a same designated work mode of the monitor and of the medical ventilation apparatus 200. The work mode key is configured to generate, based on a user operation, a control instruction which is configured to simultaneously control the monitor and the medical ventilation apparatus to enter into designated work modes. For example, the work mode key includes a rescue mode key, which is correlated with a cardiopulmonary resuscitation rescue mode of the monitor and a cardiopulmonary resuscitation ventilation mode of the medical ventilation apparatus 200. When the rescue mode key is operated, the monitor starts the cardiopulmonary resuscitation rescue mode, and the ventilation apparatus simultaneously starts the cardiopulmonary resuscitation ventilation mode, thereby reducing the number of steps to operate the apparatuses during the rescue process, allowing medical staff to focus more on patient.

In some embodiments, the controller 230 is further configured to acquire a physiological parameter which is acquired by monitoring the patient with the monitor, and the human-machine interaction interface 240 is further configured to display the acquired physiological parameter. The human-machine interaction interface 240 includes a display apparatus. In one example, the display apparatus includes at least two display areas, wherein the physiological parameter and the respiratory treatment parameter are displayed in different areas. In another example, the display apparatus is configured to fuse and display the physiological parameter and the respiratory treatment parameter. For example, the display apparatus includes multiple display areas, and different physiological parameters and different respiratory treatment parameters are displayed in different display areas. The medical ventilation apparatus 200 is also configured to fuse and display the correlated physiological parameter and respiratory treatment parameter in a same display area.

In one embodiment, the controller 230 is further configured to transmit work data of the medical ventilation apparatus to the monitor through the communication interface 250, so that the monitor can perform monitoring based on the work data of the medical ventilation apparatus, and the user can simultaneously view the work data of the medical ventilation apparatus on the monitor. For example, the work data transmitted to the monitor through the communication interface 250 is at least one of ventilation configuration data and ventilation monitoring data of the medical ventilation apparatus. After the working apparatus of the medical ventilation apparatus is transferred to the monitor, the ventilation configuration data and the ventilation monitoring data can be transferred between medical ventilation apparatuses by unplugging the monitor from one medical ventilation apparatus and then plugging the monitor into another medical ventilation apparatus.

The medical ventilation apparatus 200 in the embodiment of this disclosure communicates with the monitor through the communication interface 250. The user can control the monitor through the human-machine interaction interface 240 of the medical ventilation apparatus 200 without requiring switching apparatuses, which improves operation efficiency of the user in the monitoring and ventilation process.

A third aspect according to an embodiment of this disclosure provides a medical ventilation apparatus. Refer to FIG. 3, the medical ventilation apparatus 300 includes: a patient pipeline 310, which is connected with a patient interface, so as to deliver a ventilation gas to an airway of a patient; a pressure generator 320, which is configured to generate the ventilation gas with a preset pressure and deliver said ventilation gas to the patient; a controller 330, which is configured to control the pressure generator 320 to ventilate the patient based on configuration information which matches with the patient; a human-machine interaction interface 340, which is configured to display a respiratory treatment parameter of the medical ventilation apparatus and to acquire input information from a user; wherein the medical ventilation apparatus 300 further includes a communication interface 350, which is configured to implement information communication with a monitor; the monitor is further configured to acquire, through the communication interface 350, the physiological parameter of the patient, which parameter is measured by the monitor; wherein the human-machine interaction interface 340 is further configured to display the acquired physiological parameter.

The medical ventilation apparatus 300 in the embodiment of this disclosure is connected with the monitor through the communication interface 350. The physiological parameter acquired by the monitor can be displayed on the medical ventilation apparatus, so that the user can simultaneously view, through the medical ventilation apparatus, the respiratory treatment parameter, the input information from the user, and the physiological parameter, which are acquired by the medical ventilation apparatus.

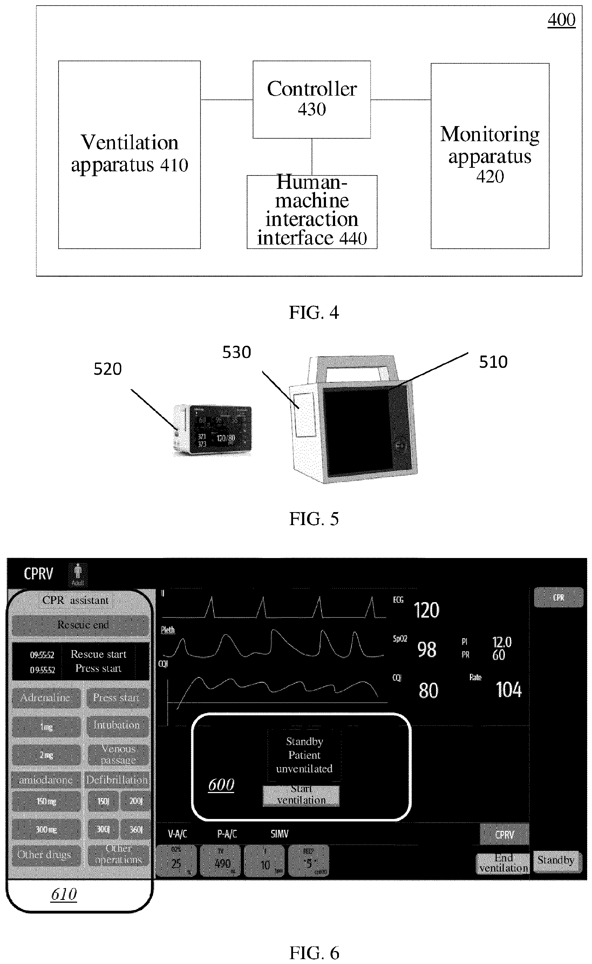

An embodiment of this disclosure further provides a medical device. Referring to FIG. 4, the medical device 400 includes a ventilation apparatus 410 and a monitoring apparatus 420, wherein the monitoring apparatus is integrated with the ventilation apparatus, where the monitoring apparatus is not capable of being dissembled from the ventilation apparatus. The ventilation apparatus 410 includes a patient pipeline, which is connected with a patient interface, so as to deliver a ventilation gas to an airway of a patient; a pressure generator, which is configured to generate the ventilation gas with a preset pressure and deliver said ventilation gas to the patient. The monitoring apparatus 420 is configured to acquire a physiological parameter of the patient through a physiological parameter sensor. The medical device 400 further includes: a controller 430, which is configured to control the ventilation apparatus 410 to ventilate the patient based on configuration information which matches with the patient, and to control the monitoring apparatus 420 to acquire a physiological parameter of the patient; and a human-machine interaction interface 440, which includes a first display screen and a second display screen; wherein the first display screen is configured to display a respiratory treatment parameter of the ventilation apparatus, and the second display screen is configured to display the physiological parameter. For example, the first display screen and the second display screen may be arranged side by side in an up-down direction.

The controller 430 may include one or more processors. For example, the ventilation apparatus 410 and the monitoring apparatus 420 may be controlled by a same processor or may be controlled by different processors.

Furthermore, the human-machine interaction interface 440 is further configured to acquire a control instruction which is inputted by the user, and the controller 430 is further configured to control the ventilation apparatus 410 to perform a first operation according to the control instruction, and to control the monitoring apparatus 420 to perform a second operation. Wherein the first operation is correlated with the second operation. Exemplarily, the first operation or the second operation includes at least one of turning on, turning off, standing by, entering into a designated work mode, and setting an alarm limit.

The medical device 400 in the embodiment of this disclosure includes a ventilation apparatus 410 and a monitoring apparatus 420, so that it can realize both a medical ventilation function and a monitoring function. Simultaneously, the user can also input a control instruction through the human-machine interaction interface 440 to collaboratively control the ventilation apparatus 410 and the monitoring apparatus 420.

An embodiment of this disclosure further provides a medical ventilation apparatus, which is in communication connection with a monitor and is configured to implement a respiratory treatment on a patient; wherein the medical ventilation apparatus includes a display apparatus, which is configured to display a respiratory treatment parameter of the medical ventilation apparatus; wherein the medical ventilation apparatus is further configured to acquire a control instruction which is inputted by a user, so as to at least control the monitor to work based on the control instruction. In one embodiment, the medical ventilation apparatus may be a ventilator for mechanically ventilating a patient, such as a portable ventilator.

Wherein, the monitor can be a portable monitor or various monitoring modules.

In one embodiment, the monitor may be a module built into the medical ventilation apparatus, or a module of the medical ventilation apparatus, which module is capable of being disassembled from the medical ventilation apparatus. As one system, the monitor and the medical ventilation apparatus can realize common power supply, one-key start/shutdown, same-screen setting/display, etc.

When the monitor is a module built into the medical ventilation apparatus, it is integrated inside the medical ventilation apparatus as a functional module of the medical ventilation apparatus and integrally-formed with the medical ventilation apparatus, but meanwhile it can realize all the functions of the monitor. The monitor may not have an independent display screen or power supply, but share the display screen and power supply with the medical ventilation apparatus. The monitor may also have an independent display screen or power supply.

In one embodiment, the monitor is built into the medical ventilation apparatus, and the monitor is not capable of being disassembled from the medical ventilation apparatus, and the display apparatus includes a first display screen and a second display screen. The first display screen is configured to display the respiratory treatment parameter of the medical ventilation apparatus, and the second display screen is configured to display the physiological parameter of the patient monitored by the monitor. The first display screen and the second display screen can be arranged side by side.

In one embodiment, when the monitor is a module built into the medical ventilation apparatus, the monitor can be assembled with a main structure inside the medical ventilation apparatus, or can be a separate module unit, which is not limited here.

In one embodiment, unlike other single-parameter measurement modules in the medical ventilation apparatus, although the monitor is a module built into the medical ventilation apparatus, it is a multi-parameter monitor, configured to monitor multiple physiological parameters, and can even have functions, such as data processing and physiological alarm.

In one embodiment, the multi-parameter monitor is at least configured to acquire an electrocardiogram parameter (ECG).

When the monitor is a module of the medical ventilation apparatus, which module is capable of being disassembled from the medical ventilation apparatus, it can be integrated with the medical ventilation apparatus, where the monitor is capable of being disassembled from the medical ventilation apparatus. At this time, the monitor can be a monitor that operates independently, for example, with an independent display apparatus and power supply. Of course, the monitor can also have no independent display apparatus and power supply, and may be powered and display through medical ventilation apparatus.

When the monitor is a module of the medical ventilation apparatus, which module is capable of being disassembled from the medical ventilation apparatus, the monitor is integrated with the medical ventilation apparatus to facilitate combination of the monitor with different medical ventilation apparatuses, wherein the monitor is capable of being disassembled from the medical ventilation apparatus. The monitor is further configured to acquire and save work data of the medical ventilation apparatus. The work data includes ventilation monitoring data and/or ventilation configuration data. When the monitor switches between different medical ventilation apparatuses, a transfer of ventilation setting and monitoring information between different medical ventilation apparatuses can be realized.

In one embodiment, when the monitor is a module of the medical ventilation apparatus, which module is capable of being disassembled from the medical ventilation apparatus, the monitor is fixed with the medical ventilation apparatus through a physical connection structure and capable of being disassembled from the medical ventilation apparatus, wherein the physical connection structure is a tangible connection structure.

In one embodiment, as shown in FIG. 5, the physical connection structure is a hot-plug structure. The medical ventilation apparatus 510 is provided with a plug-in slot 530 which supports a hot-plug monitor. The medical ventilation apparatus 510 communicates with the monitor 520 through a communication interface of the plug-in slot 530. The communication interface can be a conventional contact communication interface or an optical communication interface.

In one embodiment, the physical connection structure includes a first connection structure, which is provided at one end of the monitor which end is inserted into the plug-in slot, and a second connection structure, which is provided at a side wall of the plug-in slot, wherein the first connection structure and the second connection structure matches with each other, so as to enable the fixation of the monitor and the communication between the monitor and the medical ventilation apparatus. For example, the first connection structure and the second connection structure are locking structures.

In one embodiment, when the monitor is fixed with the medical ventilation apparatus, the monitor and the medical ventilation apparatus can also communicate through wireless means, such as Bluetooth, WIFI, etc.

The medical ventilation apparatus receives the control instruction inputted by the user, and based on the communication connection with the monitor, at least controls the work of the monitor according to the control instruction, such as controlling the monitor to turn on, off, enter into a designated work mode, etc.

In one embodiment, the control instruction is further configured to simultaneously control the work of the medical ventilation apparatus. For example, the control instruction is configured to simultaneously control the monitor and the medical ventilation apparatus to perform at least one of turning on, turning off, standing by, and entering into a designated work mode.

In one embodiment, the medical ventilation apparatus includes an interactive key, and the user inputs the control instruction by operating the interactive key.

The interactive key can be a physical key or a virtual key. For example, the interactive key is a key provided at the medical ventilation apparatus, or the interactive key is a control provided on a display interface of the display apparatus. There is no limitation here, as if the control instruction can be generated based on the operation of the user.

In one embodiment, in addition to providing the interactive key, the monitor and the medical ventilation apparatus can also be provided with separate control keys, so as to independently control the monitor and the medical ventilation apparatus, respectively. For example, the monitor includes a monitoring switch, which is configured to individually control turning on, turning off, standing by, etc., of the monitor; the medical ventilation apparatus includes a ventilation switch, which is configured to individually control turning on, turning off, ventilation starting, standing by, etc., of the ventilation apparatus.

In one embodiment, the interactive key includes a work mode key, which is correlated with a same work mode of the monitor and of the medical ventilation apparatus, and the work mode key is configured to generate, based on a user operation, a control instruction which is configured to simultaneously control the monitor and the medical ventilation apparatus to enter into designated work modes.

In one embodiment, the work mode key includes a rescue mode key, which is respectively correlated with a cardiopulmonary resuscitation rescue mode of the monitor and a cardiopulmonary resuscitation ventilation mode of the medical ventilation apparatus. When the rescue mode key is operated based on the operation of the user, the rescue mode key generates a corresponding control instruction, so as to control the monitor to start the cardiopulmonary resuscitation rescue mode, and simultaneously control the medical ventilation apparatus to start the cardiopulmonary resuscitation ventilation mode. When the monitor starts the cardiopulmonary resuscitation rescue mode, for example, it can start a rescue assistant unit to record a rescue process, and start an effect evaluation unit to evaluate an effect of a cardiopulmonary resuscitation rescue.

In one embodiment, when the rescue mode key is operated, if the medical ventilation apparatus is in a ventilation treatment state (non-cardiopulmonary resuscitation ventilation mode), the ventilation mode is switched to the cardiopulmonary resuscitation ventilation mode and kept in a standby state, and the ventilation of the medical ventilation apparatus is restarted after the user controls to start the ventilation. If the medical ventilation apparatus is in a standby mode, the medical ventilation apparatus is controlled to enter into the cardiopulmonary resuscitation ventilation mode, and the ventilation of the medical ventilation apparatus is started directly.

In one embodiment, the rescue assistant unit is configured to record at least one of following matters during the rescue process:
start time and end time of a rescue, name(s) and/or dose(s) of drug(s) used during the rescue, and operation(s) performed during the rescue.

In one embodiment, the rescue assistant unit is configured to output a record of the rescue process and form a rescue report.

Wherein, the effect evaluation unit is configured to display waveform(s) and/or monitoring parameter(s) of at least one of electrocardiogram, blood oxygen saturation and carbon dioxide of a monitored subject in the cardiopulmonary resuscitation rescue mode, and/or is configured to display a continuous quality improvement parameter.

In another embodiment, when the rescue mode key is operated, the monitor is controlled to start the rescue assistant unit for recording the rescue process, and the monitor is controlled to start the effect evaluation unit for evaluating the cardiopulmonary resuscitation effect. Simultaneously the medical ventilation apparatus is controlled to display the cardiopulmonary resuscitation ventilation mode, and the user manually controls the medical ventilation apparatus to start ventilation under the cardiopulmonary resuscitation ventilation mode.

In one embodiment, the medical ventilation apparatus is further configured to acquire the physiological parameter of the patient monitored by the monitor.

The medical ventilation apparatus includes a display apparatus for displaying the respiratory treatment parameter of the medical ventilation apparatus and the acquired physiological parameter from the monitor.

The display apparatus may include multiple display areas to display different physiological parameters, respectively. For example, in one embodiment, the display apparatus includes at least two display areas, and the physiological parameter and the respiratory treatment parameter are displayed in different areas of the display apparatus. For example, different physiological parameters and different respiratory treatment parameters are displayed in different display areas. It should be noted that the two display areas may be formed by two independent display screens, or may be formed by the same display screen.

In one embodiment, the display area may be a display window. For example, the display apparatus includes a first display window and a second display window to respectively display the physiological parameter and the respiratory treatment parameter.

In one embodiment, the display apparatus is further configured to fuse and display the physiological parameter and the respiratory treatment parameter. For example, the medical ventilation apparatus is further configured to correlate the monitoring parameter with the respiratory treatment parameter, which parameters have the same type. The medical ventilation apparatus is further configured to fuse and display, in the same display area, the monitoring parameter and the respiratory treatment parameter which are correlated with each other.

For example, in one embodiment, the medical ventilation apparatus establishes a correlation between its internal respiratory treatment parameter in the cardiopulmonary resuscitation ventilation mode and the physiological parameter in the cardiopulmonary resuscitation rescue mode of the monitor, and displays in the same display area, the respiratory treatment parameter in the cardiopulmonary resuscitation ventilation mode of the medical ventilation apparatus and the physiological parameter in the cardiopulmonary resuscitation rescue mode of the monitor, based on the correlation, or fuses the relevant respiratory treatment parameter into waveform(s) of the physiological parameter for display.

The medical ventilation apparatus may further include another human-machine interaction apparatus, in addition to the display apparatus, for receiving user operation and generating corresponding control instruction, so as to control the medical ventilation apparatus and the monitor. The human-machine interaction apparatus may include an input device for detecting input information from the user. The input device may include one or a combination of a keyboard, a mouse, a scroll wheel, a trackball, a mobile input device (such as a mobile device with a touch display screen, a mobile phone, etc.), a multi-function knob, etc. The human-machine interaction apparatus may also include an output device, such as a printer.

The medical ventilation apparatus may also include a memory, which is configured to store the control instruction, the physiological parameter, the respiratory treatment parameter, and the information recorded by the rescue assistant unit during the rescue process. The memory can be a flash memory card, a solid-state memory, a hard disk, etc. The memory can also be a volatile memory and/or a non-volatile memory, or a removable memory and/or a non-removable memory.

According to the embodiment of this disclosure, the medical ventilation apparatus is in communication connection with the monitor. The medical ventilation apparatus is further configured to acquire the control instruction which is inputted by the user, and at least control the work of the monitor based on the control instruction. The medical ventilation apparatus can control the monitor, so as to improve the operation efficiency during ventilation and monitoring and reduce the workload of medical staff.

Embodiments of this disclosure further provide a medical ventilation apparatus, which is in communication connection with a monitor and is configured to implement a respiratory treatment on a patient. The medical ventilation apparatus includes a display apparatus, which is configured to display a respiratory treatment parameter of the medical ventilation apparatus; wherein the medical ventilation apparatus is further configured to acquire a physiological parameter of the patient, which parameter is monitored by the monitor, and to display the physiological parameter through the display apparatus.

It should be noted that the medical ventilation apparatus introduced in the following embodiments can adopt solutions mentioned in the above corresponding embodiments, and therefore these solutions are described in detail.

In one embodiment, the monitor and the medical ventilation apparatus are in wireless communication connection with each other; or the monitor is fixed with the medical ventilation apparatus through a physical connection structure and communicates with the medical ventilation apparatus, wherein the monitor is capable of being disassembled from the medical ventilation apparatus; or the monitor is a built-in module of the medical ventilation apparatus.

When the monitor is a module built into the medical ventilation apparatus, it is integrated inside the medical ventilation apparatus as a functional module of the medical ventilation apparatus and integrally-formed with the medical ventilation apparatus, but meanwhile it can realize all the functions of the monitor. The monitor may not have an independent display screen or power supply, and share the display screen and power supply with the medical ventilation apparatus. The monitor may also have an independent display screen or power supply.

In one embodiment, the monitor is built into the medical ventilation apparatus and the monitor is not capable of being disassembled from the medical ventilation apparatus, and the display apparatus includes a first display screen and a second display screen. The first display screen is configured to display the respiratory treatment parameter of the medical ventilation apparatus, and the second display screen is configured to display the physiological parameter of the patient monitored by the monitor. The first display screen and the second display screen can be arranged side by side.

It should be noted that, unlike other single-parameter measurement modules in the medical ventilation apparatus, although the monitor is a module built into the medical ventilation apparatus, it is a multi-parameter monitor, configured to monitor multiple physiological parameters, and can even have functions, such as data processing and physiological alarm, etc.

In one embodiment, unlike other single-parameter measurement modules in the medical ventilation apparatus, although the monitor is a module built into the medical ventilation apparatus, it is a multi-parameter monitor, configured to monitor multiple physiological parameters, and can even have functions, such as data processing and physiological alarm, etc.

In one embodiment, the multi-parameter monitor is at least configured to acquire an electrocardiogram parameter (ECG).

When the monitor is a module of the medical ventilation apparatus, which module is capable of being disassembled from the medical ventilation apparatus, it can be integrated with the medical ventilation apparatus, where the monitor is capable of being dissembled from the ventilation apparatus. At this time, the monitor can be a monitor that operates independently, for example, with an independent display apparatus and power supply. Of course, the monitor can also have no independent display apparatus and power supply, and may be powered and display through medical ventilation apparatus.

When the monitor is a module of the medical ventilation apparatus, which module is capable of being disassembled from the medical ventilation apparatus, the monitor is integrated with the medical ventilation apparatus to facilitate combination of the monitor with different medical ventilation apparatuses, where the monitor is capable of being dissembled from the ventilation apparatus. The monitor is further configured to acquire and save work data of the medical ventilation apparatus. The work data includes ventilation monitoring data and/or ventilation configuration data. When the monitor switches between different medical ventilation apparatuses, a transfer of ventilation setting and monitoring information between different medical ventilation apparatuses can be realized.

In one embodiment, when the monitor is a module of the medical ventilation apparatus, which module is capable of being disassembled from the medical ventilation apparatus, the monitor is fixed with the medical ventilation apparatus through a physical connection structure and capable of being disassembled from the medical ventilation apparatus, wherein the physical connection structure is a tangible connection structure.

In one embodiment, the physical connection structure is a hot-plug structure, and the medical ventilation apparatus is provided with a plug-in slot which supports a hot-plug monitor. The medical ventilation apparatus acquires the physiological parameter of the patient monitored by the monitor through the communication interface of the plug-in slot. The communication interface can be a conventional contact communication interface or an optical communication interface.

In one embodiment, when the monitor is fixed with the medical ventilation apparatus, the monitor and the medical ventilation apparatus can also communicate with each other through wireless means, such as Bluetooth, WIFI, etc.

In one embodiment, the physical connection structure includes a first connection structure, which is provided on one end of the monitor which end is inserted into the plug-in slot, and a second connection structure, which is provided at a side wall of the plug-in slot, wherein the first connection structure and the second connection structure matches with each other. For example, the first connection structure and the second connection structure are locking structures.

In one embodiment, the display apparatus includes at least two display areas, wherein the physiological parameter and the respiratory treatment parameter are displayed in different areas.

In one embodiment, the display apparatus is further configured to fuse and display the physiological parameter and the respiratory treatment parameter.

In one embodiment, the display apparatus includes multiple display areas, and different physiological parameters and different respiratory treatment parameters are displayed in different display areas. The medical ventilation apparatus is further configured to fuse and display correlated physiological parameter and respiratory treatment parameter within the same display area.

In one embodiment, the medical ventilation apparatus includes a portable ventilator.

In one embodiment, the monitor is further configured to acquire and save work data of the medical ventilation apparatus. Specifically, the work data may include ventilation configuration data and ventilation monitoring data of the medical ventilation apparatus.

Embodiment of this disclosure further provide a medical ventilation apparatus, which is in communication connection with a monitor. The medical ventilation apparatus is configured to implement a respiratory treatment on the patient. The monitor is configured to monitor the physiological parameter of the patient; the medical ventilation apparatus is configured to acquire a control instruction which is inputted by the user, and to control the monitor to enter a CPR rescue mode based on the control instruction.

In one embodiment, the medical ventilation apparatus includes a display apparatus. The medical ventilation apparatus is configured to control the display apparatus to display the respiratory treatment parameter of the medical ventilation apparatus, acquire the physiological parameter of the patient monitored by the monitor, and control the display apparatus to display the physiological parameter.

In one embodiment, the medical ventilation apparatus is further configured to control the display apparatus to display a CPR specific tool interface, which is configured to display CPR-related information.

In one embodiment, the CPR specific tool interface is provided with a rescue assistant unit, which is configured to record a rescue process; and an effect evaluation unit, which is configured to evaluate a rescue effect of cardiopulmonary resuscitation.

The rescue assistant unit is configured to record the rescue process. It can record start time and end time of a rescue, name(s) and/or dose(s) of drug(s) used during the rescue, and operation(s) performed during the rescue, etc. After the rescue is completed, the rescue record can be output through a recorder, or a rescue report can be output through a printer.

The effect evaluation unit displays waveforms and monitoring parameters of key physiological parameters, such as ECG, SpO₂, and CO₂ of the patient during the CPR rescue process. It can also display the continuous quality improvement parameter (CQI parameter), which is an important parameter for evaluating the effect of cardiopulmonary resuscitation for the patient.

In one embodiment, the medical ventilation apparatus is further configured to enter a CPRV ventilation mode based on the control instruction to ventilate the patient. The CPRV mode is the cardiopulmonary resuscitation ventilation mode. It is a ventilation mode used during the cardiopulmonary resuscitation compression process. It can quickly start during the cardiopulmonary resuscitation compression process, provide timely mechanical ventilation support to the patient, and effectively avoid excessive pressure during the cardiopulmonary resuscitation compression process, and meanwhile avoid harm to patient caused by frequent triggering and hyperventilation during the cardiopulmonary resuscitation compression process.

In one embodiment, as shown in area 600 of FIG. 6, when the patient is in an early stage of CPR rescue and an artificial airway has not yet been established, the machine is in a standby mode. At this time, a "rescue mode" key is pressed, as shown in area 610 in FIG.6, the CPR monitoring-related functions (CPR assistant + CPR process monitoring and cardiopulmonary resuscitation effect evaluation) can be started firstly. The ventilator has not yet started ventilation and is in the standby mode. However, the ventilation mode is switched to the CPRV mode at this time, and the doctor can manually start CPRV ventilation through the "Start Ventilation" key shown in the interface area 600 .

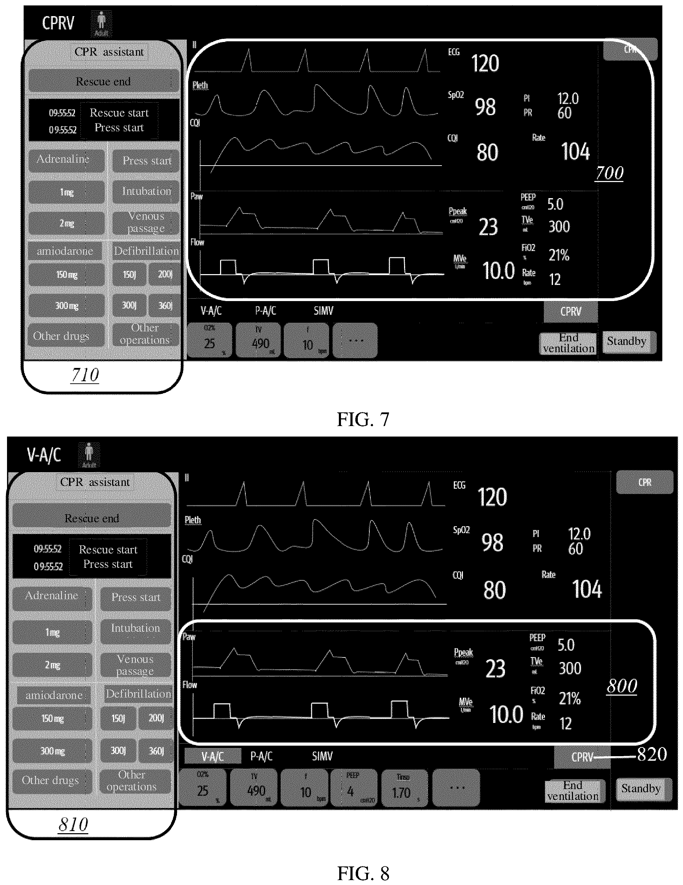

In one embodiment, as shown in FIG. 7, the patient suffers cardiac arrest during a ventilation and monitoring process and requires a CPR rescue. At this time, press the "rescue mode" key to start CPR monitoring-related functions (CPR assistant + CPR process monitoring and cardiopulmonary resuscitation effect evaluation) and CPRV ventilation therapy, as shown in area 710 and area 700.

In one embodiment, as shown in FIG. 8, the patient suffers cardiac arrest during a ventilation and monitoring process and requires a CPR rescue. CPR monitoring-related functions and CPRV ventilation are started separately. That is, press the "rescue mode" key, as shown in area 810, to start CPR monitoring-related functions (CPR Assistant + CPR process monitoring and cardiopulmonary resuscitation effect evaluation), while the current ventilation mode remains unchanged, as shown in area 800. If it is needed to start the CPRV ventilation mode, the user manually selects the CPRV mode 820 and confirms to switch the ventilation mode.

It should be noted that in FIGS. 6 to 8, underlined labels 600, 610, 700, 710, 800, 810 and the corresponding rounded rectangles are only configured to identify the corresponding areas and should not be understood as the content displayed by the display apparatus.

In one embodiment, the monitor and the medical ventilation apparatus are in wireless communication connection with each other; or the monitor is fixed with the medical ventilation apparatus through a physical connection structure and communicates with the medical ventilation apparatus, wherein the monitor is capable of being disassembled from the medical ventilation apparatus; or the monitor is a built-in module of the medical ventilation apparatus.

In one embodiment, the physical connection structure is a hot-plug structure, the medical ventilation apparatus is provided with a plug-in slot which supports a hot-plug monitor, and the medical ventilation apparatus communicates with the monitor through a communication interface of the plug-in slot.

In one embodiment, the monitor is a module of the medical ventilation apparatus, which module is capable of being disassembled from the medical ventilation apparatus.

By correlating different functions and modes with each other, and quickly realizing operations in a specific mode (such as CPRV mode) based on the correlation, the medical ventilation apparatus can improve the operation efficiency during ventilation and monitoring, reduce the workload of medical staff, and improve the effectiveness of patient treatment in emergency situation.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some characteristics can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Ordinary technical personnel in this field can understand that, in addition to mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

In addition, Ordinary technical personnel in this field can understand that although some embodiments described herein include certain characteristics rather than other characteristics included in other embodiments, the combination of characteristics of different embodiments means that they are within the scope of this disclosure and form different embodiments. For example, in the claims, any one of the claimed embodiments can be used in any combination.

The various component embodiments of this disclosure can be implemented in hardware, software modules running on one or more processors, or a combination thereof. Ordinary technical personnel in this field should understand that a microprocessor or digital signal processor (DSP) can be used in practice to implement some or all of the functions of some modules according to the embodiments of this disclosure. This disclosure can also be implemented as a partial or complete device program (such as a computer program and a computer program product) for executing the method described herein. The program implementing this disclosure can be stored at a computer-readable medium or can have the form of one or more signals. Such signals can be downloaded from internet websites, provided on carrier signals, or in any other form.

It should be noted that the above embodiments illustrate this disclosure rather than limit it, and Ordinary technical personnel in this field can design alternative embodiments without departing from the scope of the attached claims. In the claims, any reference symbol between parentheses should not be constructed as a restriction on the claims. This disclosure can be implemented with the help of hardware consisting of several different components and with the help of appropriately programmed computers. Among the unit claims that list several devices, several of these devices can be specifically embodied through the same hardware item. The use of words first, second, and third does not indicate any order. These words can be interpreted as names.

The above is only the specific implementation method or explanation of the specific implementation method of this disclosure. The protection scope of this disclosure is not limited to this. Any technical personnel familiar with the technical field can easily think of changes or replacements within the technical scope disclosed in this disclosure, and those changes or replacements should fall into the protection scope of this disclosure. The protection scope of this disclosure shall be based on the protection scope of the claims.

## Claims

1. A medical ventilation apparatus, **characterized in that**, comprising:
a patient pipeline, which is connected with a patient interface, so as to deliver a ventilation gas to an airway of a patient;
a pressure generator, which is configured to generate the ventilation gas with a preset pressure and deliver said ventilation gas to the patient;
a controller, which is configured to control the pressure generator to ventilate the patient based on configuration information which matches with the patient; and
a human-machine interaction interface, which is configured to display a respiratory treatment parameter of the medical ventilation apparatus and to acquire input information from a user;
wherein the medical ventilation apparatus is further provided with a physical connection structure, which is configured to assemble a monitor with the medical ventilation apparatus, which assembly is capable of being disassembled; wherein the monitor is configured to measure a physiological parameter of the patient, when the medical ventilation apparatus implements a ventilation treatment on the patient;
wherein the medical ventilation apparatus further comprises a communication interface, which is configured to implement information communication with the monitor, when the monitor is assembled with the medical ventilation apparatus.

2. The medical ventilation apparatus according to claim 1, **characterized in that**, the human-machine interaction interface is further configured to acquire a control instruction which is inputted by the user;
the controller is further configured to transmit the control instruction to the monitor through the communication interface according to the control instruction, so as to control the monitor to implement a first operation.

3. The medical ventilation apparatus according to claim 2, **characterized in that**, the controller is further configured to control the medical ventilation apparatus to implement a second operation according to the control instruction, and the first operation is correlated with the second operation.

4. The medical ventilation apparatus according to claim 3, **characterized in that**, the first operation or the second operation comprises at least one of turning on, turning off, standing by, entering into a designated work mode, and setting an alarm limit.

5. The medical ventilation apparatus according to claim 4, **characterized in that**, the human-machine interaction interface comprises a work mode key, which is correlated with a corresponding designated work mode of the monitor and of the medical ventilation apparatus; wherein the work mode key is configured to generate, based on an operation of the user, a control instruction which is configured to simultaneously control the monitor and the medical ventilation apparatus to enter into said designated work mode.

6. The medical ventilation apparatus according to claim 5, **characterized in that**, the work mode key comprises a rescue mode key, which is correlated with a cardiopulmonary resuscitation rescue mode of the monitor and a cardiopulmonary resuscitation ventilation mode of the medical ventilation apparatus;
wherein, when determining that the user operates the rescue mode key, the monitor starts the cardiopulmonary resuscitation rescue mode, and the medical ventilation apparatus simultaneously starts the cardiopulmonary resuscitation ventilation mode.

7. The medical ventilation apparatus according to any one of claims 1-6, **characterized in that**, the controller is further configured to acquire the physiological parameter of the patient, which parameter is monitored by the monitor, and the human-machine interaction interface is further configured to display the acquired physiological parameter.

8. The medical ventilation apparatus according to claim 7, **characterized in that**, the human-machine interaction interface comprises a display apparatus, wherein the display apparatus comprises at least two display areas, the physiological parameter and the respiratory treatment parameter are displayed in different display areas.

9. The medical ventilation apparatus according to claim 7, **characterized in that**, the human-machine interaction interface comprises a display apparatus, which is configured to fuse and display the physiological parameter and the respiratory treatment parameter.

10. The medical ventilation apparatus according to claim 9, **characterized in that**, the display apparatus comprises multiple display areas, wherein different physiological parameters and different respiratory treatment parameters are displayed in different display areas;
the medical ventilation apparatus is further configured to fuse and display, in a same display area, the physiological parameter and the respiratory treatment parameter which are correlated with each other.

11. The medical ventilation apparatus according to any one of claims 1-10, **characterized in that**, the physical connection structure comprises a hot-plug structure, and the medical ventilation apparatus is further provided with a plug-in slot which supports hot-plugging of the monitor; wherein the medical ventilation apparatus communicates with the monitor through the communication interface of the plug-in slot.

12. The medical ventilation apparatus according to any one of claims 1-11, **characterized in that**, the human-machine interaction interface comprises a touch display screen.

13. The medical ventilation apparatus according to any one of claims 1-12, **characterized in that**, the communication interface comprises at least one of a contact communication interface, an optical communication interface, a Bluetooth communication interface, and a WIFI communication interface.

14. The medical ventilation apparatus according to any one of claims 1-13, **characterized in that**, further comprising a power supply apparatus and a common power interface; wherein, when the monitor is assembled with the medical ventilation apparatus, the power supply apparatus is configured to simultaneously supply power to the medical ventilation apparatus and the monitor.

15. The medical ventilation apparatus according to any one of claims 1-14, **characterized in that**, the monitor is a multi-parameter monitor.

16. The medical ventilation apparatus according to claim 15, **characterized in that**, a plurality of physiological parameters, which are acquired by the multi-parameter monitor, comprise an ECG parameter.

17. The medical ventilation apparatus according to any one of claims 1-16, **characterized in that**, the medical ventilation apparatus comprises a portable ventilator, and the monitor comprises a transport monitor.

18. The medical ventilation apparatus according to any one of claims 1-17, **characterized in that**, the controller is further configured to transmit work data of the medical ventilation apparatus to the monitor through the communication interface.

19. The medical ventilation apparatus according to claim 18, **characterized in that**, the work data is ventilation configuration data and/or ventilation monitoring data of the medical ventilation apparatus.

20. A medical ventilation apparatus, **characterized in that**, comprising:
a patient pipeline, which is connected with a patient interface, so as to deliver a ventilation gas to an airway of a patient;
a pressure generator, which is configured to generate the ventilation gas with a preset pressure and deliver said ventilation gas to the patient;
a controller, which is configured to control the pressure generator to ventilate the patient based on configuration information which matches with the patient; and
a human-machine interaction interface, which is configured to display a respiratory treatment parameter of the medical ventilation apparatus and to acquire input information from a user;
wherein the medical ventilation apparatus further comprises a communication interface, which is configured to implement information communication with a monitor; wherein the monitor is configured to measure a physiological parameter of the patient, when the medical ventilation apparatus implements a ventilation treatment on the patient;
wherein the human-machine interaction interface is further configured to acquire a control instruction which is inputted by the user, and the controller is further configured to transmit the control instruction to the monitor through the communication interface according to the control instruction, so as to control the monitor to implement a first operation.

21. The medical ventilation apparatus according to claim 20, **characterized in that**, the controller is further configured to control the medical ventilation apparatus to implement a second operation according to the control instruction, and the first operation is correlated with the second operation.

22. The medical ventilation apparatus according to claim 21, **characterized in that**, the first operation or the second operation comprises at least one of turning on, turning off, standing by, entering into a designated work mode, and setting an alarm limit.

23. The medical ventilation apparatus according to claim 22, **characterized in that**, the human-machine interaction interface comprises a work mode key, which is correlated with a same designated work mode of the monitor and of the medical ventilation apparatus; wherein the work mode key is configured to generate, based on an operation of the user, a control instruction which is configured to simultaneously control the monitor and the medical ventilation apparatus to enter into said designated work mode.

24. The medical ventilation apparatus according to claim 23, **characterized in that**, the work mode key comprises a rescue mode key, which is correlated with a cardiopulmonary resuscitation rescue mode of the monitor and a cardiopulmonary resuscitation ventilation mode of the medical ventilation apparatus;
wherein, when the rescue mode key is operated, the monitor starts the cardiopulmonary resuscitation rescue mode, and the medical ventilation apparatus simultaneously starts the cardiopulmonary resuscitation ventilation mode.

25. The medical ventilation apparatus according to any one of claims 20-24, **characterized in that**, the controller is further configured to acquire the physiological parameter of the patient, which parameter is monitored by the monitor, and the human-machine interaction interface is further configured to display the acquired physiological parameter.

26. The medical ventilation apparatus according to claim 25, **characterized in that**, the human-machine interaction interface comprises a display apparatus, wherein the display apparatus comprises at least two display areas, the physiological parameter and the respiratory treatment parameter are displayed in different display areas.

27. The medical ventilation apparatus according to claim 25, **characterized in that**, the human-machine interaction interface comprises a display apparatus, which is configured to fuse and display the physiological parameter and the respiratory treatment parameter.

28. The medical ventilation apparatus according to claim 27, **characterized in that**, the display apparatus comprises multiple display areas, wherein different physiological parameters and different respiratory treatment parameters are displayed in different display areas;
the medical ventilation apparatus is further configured to fuse and display, in a same display area, the physiological parameter and the respiratory treatment parameter which are correlated with each other.

29. The medical ventilation apparatus according to any one of claims 20-28, **characterized in that**, the monitor is a multi-parameter monitor.

30. The medical ventilation apparatus according to claim 29, **characterized in that**, the multi-parameter monitor is at least configured to acquire an ECG parameter.

31. The medical ventilation apparatus according to any one of claims 20-30, **characterized in that**, the medical ventilation apparatus comprises a portable ventilator.

32. The medical ventilation apparatus according to any one of claims 20-31, **characterized in that**, the controller is further configured to transmit work data of the medical ventilation apparatus to the monitor through the communication interface.

33. The medical ventilation apparatus according to claim 32, **characterized in that**, the work data is ventilation configuration data or ventilation monitoring data of the medical ventilation apparatus.

34. A medical ventilation apparatus, **characterized in that**, comprising:
a patient pipeline, which is connected with a patient interface, so as to deliver a ventilation gas to an airway of a patient;
a pressure generator, which is configured to generate the ventilation gas with a preset pressure and deliver said ventilation gas to the patient;
a controller, which is configured to control the pressure generator to ventilate the patient based on configuration information which matches with the patient; and
a human-machine interaction interface, which is configured to display a respiratory treatment parameter of the medical ventilation apparatus and to acquire input information from a user;
wherein the medical ventilation apparatus further comprises a communication interface, which is configured to implement information communication with a monitor; wherein the monitor is configured to measure a physiological parameter of the patient, when the medical ventilation apparatus implements a ventilation treatment on the patient;
wherein the controller is further configured to acquire, through the communication interface, the physiological parameter of the patient, which parameter is measured by the monitor; the human-machine interaction interface is further configured to display the acquired physiological parameter.

35. A medical device, **characterized in that**, comprising: a ventilation apparatus and a monitoring apparatus; wherein the monitoring apparatus is integrated with the ventilation apparatus where the monitoring apparatus is not capable of being dissembled from the ventilation apparatus;
wherein the ventilation apparatus comprises:
a patient pipeline, which is connected with a patient interface, so as to deliver a ventilation gas to an airway of a patient; and
a pressure generator, which is configured to generate the ventilation gas with a preset pressure and deliver said ventilation gas to the patient;
wherein the monitoring apparatus is configured to acquire a physiological parameter of the patient through a physiological parameter sensor;
wherein the medical device further comprises:
a controller, which is configured to control the ventilation apparatus to ventilate the patient based on configuration information which matches with the patient, and configured to control the monitoring apparatus to acquire the physiological parameter of the patient; and
a human-machine interaction interface, which comprises a first display screen and a second display screen, wherein the first display screen is configured to display a respiratory treatment parameter of the ventilation apparatus, the second display screen is configured to display the physiological parameter.

36. The medical device according to claim 35, **characterized in that**, the human-machine interaction interface is further configured to acquire a control instruction which is inputted by a user;
the controller is further configured to control the ventilation apparatus to implement a first operation according to the control instruction, and to control the monitoring apparatus to implement a second operation according to the control instruction, wherein the first operation is correlated with the second operation.

37. The medical device according to claim 36, **characterized in that**, the first operation or the second operation comprises at least one of turning on, turning off, standing by, entering into a designated work mode, and setting an alarm limit.

38. A medical ventilation apparatus, which is in communication connection with a monitor and is configured to implement a respiratory treatment on a patient; wherein the medical ventilation apparatus comprises a display apparatus, which is configured to display a respiratory treatment parameter of the medical ventilation apparatus; **characterized in that**, the medical ventilation apparatus is further configured to acquire a control instruction which is inputted by a user, so as to at least control the monitor to work based on the control instruction.

39. The medical ventilation apparatus according to claim 38, **characterized in that**, in order to at least control the monitor to work based on the control instruction, the medical ventilation apparatus is further configured to simultaneously control, based on the control instruction, the monitor and the medical ventilation apparatus to implement at least one of turning on, turning off, standing by, entering into a designated work mode, and setting an alarm limit.

40. The medical ventilation apparatus according to claim 39, **characterized in that**, the medical ventilation apparatus comprises an interactive key, which key is configured to generate the control instruction based on an operation of the user.

41. The medical ventilation apparatus according to claim 40, **characterized in that**, the interactive key comprises a work mode key, which is correlated with a same designated work mode of the monitor and of the medical ventilation apparatus; wherein the work mode key is configured to generate, based on the operation of the user, a control instruction which is configured to simultaneously control the monitor and the medical ventilation apparatus to enter into said designated work mode.

42. The medical ventilation apparatus according to claim 41, **characterized in that**, the work mode key comprises a rescue mode key, which is correlated with a cardiopulmonary resuscitation rescue mode of the monitor and a cardiopulmonary resuscitation ventilation mode of the medical ventilation apparatus;
wherein, when the rescue mode key is operated, the monitor starts the cardiopulmonary resuscitation rescue mode, and the medical ventilation apparatus simultaneously starts the cardiopulmonary resuscitation ventilation mode.

43. The medical ventilation apparatus according to claim 38, **characterized in that**, the monitor and the medical ventilation apparatus are in wireless communication connection with each other; or the monitor is fixed with the medical ventilation apparatus through a physical connection structure and communicates with the medical ventilation apparatus, wherein the monitor is capable of being disassembled from the medical ventilation apparatus; or the monitor is a built-in module of the medical ventilation apparatus.

44. The medical ventilation apparatus according to claim 43, **characterized in that**, the physical connection structure is a hot-plug structure, and the medical ventilation apparatus is provided with a plug-in slot which supports hot-plugging of the monitor; wherein the medical ventilation apparatus communicates with the monitor through a communication interface of the plug-in slot.

45. The medical ventilation apparatus according to claim 38, **characterized in that**, the monitor is a module of the medical ventilation apparatus, which module is capable of being disassembled from the medical ventilation apparatus.

46. The medical ventilation apparatus according to any one of claims 38-45, **characterized in that**, the medical ventilation apparatus is further configured to acquire a physiological parameter of the patient, which parameter is monitored by the monitor; wherein the display apparatus is further configured to display the respiratory treatment parameter of the medical ventilation apparatus and the acquired physiological parameter.

47. The medical ventilation apparatus according to claim 46, **characterized in that**, the display apparatus comprises at least two display areas, the physiological parameter and the respiratory treatment parameter are displayed in different display areas.

48. The medical ventilation apparatus according to claim 46, **characterized in that**, the display apparatus is further configured to fuse and display the physiological parameter and the respiratory treatment parameter.

49. The medical ventilation apparatus according to claim 48, **characterized in that**, the display apparatus comprises multiple display areas, wherein different physiological parameters and different respiratory treatment parameters are displayed in different display areas;
the medical ventilation apparatus is further configured to fuse and display, in a same display area, the physiological parameter and the respiratory treatment parameter which are correlated with each other.

50. A medical ventilation apparatus, which is in communication connection with a monitor and is configured to implement a respiratory treatment on a patient; wherein the medical ventilation apparatus comprises a display apparatus, which is configured to display a respiratory treatment parameter of the medical ventilation apparatus; **characterized in that**, the medical ventilation apparatus is further configured to acquire a physiological parameter of the patient, which parameter is monitored by the monitor, and to display the physiological parameter through the display apparatus.

51. The medical ventilation apparatus according to claim 50, **characterized in that**, the monitor and the medical ventilation apparatus are in wireless communication connection with each other; or the monitor is fixed with the medical ventilation apparatus through a physical connection structure and communicates with the medical ventilation apparatus, wherein the monitor is capable of being disassembled from the medical ventilation apparatus; or the monitor is a built-in module of the medical ventilation apparatus.

52. The medical ventilation apparatus according to claim 50, **characterized in that**, the monitor is a multi-parameter monitor.

53. The medical ventilation apparatus according to claim 50, **characterized in that**, the multi-parameter monitor is at least configured to acquire an ECG parameter.

54. The medical ventilation apparatus according to claim 51, **characterized in that**, the physical connection structure is a hot-plug structure, and the medical ventilation apparatus is provided with a plug-in slot which supports hot-plugging of the monitor; wherein the medical ventilation apparatus is further configured to acquire, through a communication interface of the plug-in slot, the physiological parameter of the patient, which parameter is monitored by the monitor.

55. The medical ventilation apparatus according to claim 50, **characterized in that**, the monitor is a module of the medical ventilation apparatus, which module is capable of being disassembled from the medical ventilation apparatus.

56. The medical ventilation apparatus according to claim 55, **characterized in that**, the physical connection structure comprises a first connection structure which is provided at one end of the monitor, which end is inserted into a plug-in slot, and a second connection structure which is provided at a side wall of the plug-in slot, wherein the first connection structure and the second connection structure matches with each other.

57. The medical ventilation apparatus according to any one of claims 50-56, **characterized in that**, the display apparatus comprises at least two display areas, the physiological parameter and the respiratory treatment parameter are displayed in different display areas.

58. The medical ventilation apparatus according to any one of claims 50-56, **characterized in that**, the display apparatus is further configured to fuse and display the physiological parameter and the respiratory treatment parameter.

59. The medical ventilation apparatus according to claim 58, **characterized in that**, the display apparatus comprises multiple display areas, wherein different physiological parameters and different respiratory treatment parameters are displayed in different display areas;
the medical ventilation apparatus is further configured to fuse and display, in a same display area, the physiological parameter and the respiratory treatment parameter which are correlated with each other.

60. The medical ventilation apparatus according to claim 50, **characterized in that**, the medical ventilation apparatus comprises a portable ventilator.

61. The medical ventilation apparatus according to any one of claims 50-60, **characterized in that**, the monitor is further configured to acquire and save work data of the medical ventilation apparatus.

62. A medical ventilation apparatus, which is in communication connection with a monitor and is configured to implement a respiratory treatment on a patient; wherein the monitor is configured to monitor a physiological parameter of the patient; **characterized in that**, the medical ventilation apparatus is further configured to acquire a control instruction which is inputted by a user, and to control the monitor to enter into a CPR rescue mode based on the control instruction.

63. The medical ventilation apparatus according to claim 62, **characterized in that**, the medical ventilation apparatus comprises a display apparatus, and the medical ventilation apparatus is further configured to control the display apparatus to display a respiratory treatment parameter of the medical ventilation apparatus, to acquire the physiological parameter of the patient, which parameter is monitored by the monitor, and to control the display apparatus to display the physiological parameter.

64. The medical ventilation apparatus according to claim 63, **characterized in that**, the medical ventilation apparatus is further configured to control the display apparatus to display a CPR-specific tool interface, which interface is configured to display CPR-related information.

65. The medical ventilation apparatus according to claim 62, **characterized in that**, the medical ventilation apparatus is further configured to enter into a CPR ventilation mode based on the control instruction, so as to ventilate the patient.

66. The medical ventilation apparatus according to claim 62, **characterized in that**, the monitor and the medical ventilation apparatus are in wireless communication connection with each other; or the monitor is fixed with the medical ventilation apparatus through a physical connection structure and communicates with the medical ventilation apparatus, wherein the monitor is capable of being disassembled from the medical ventilation apparatus; or the monitor is a built-in module of the medical ventilation apparatus.

67. The medical ventilation apparatus according to claim 66, **characterized in that**, the physical connection structure is a hot-plug structure, and the medical ventilation apparatus is provided with a plug-in slot which supports hot-plugging of the monitor; wherein the medical ventilation apparatus communicates with the monitor through a communication interface of the plug-in slot.

68. The medical ventilation apparatus according to claim 62, **characterized in that**, the monitor is a module of the medical ventilation apparatus, which module is capable of being disassembled from the medical ventilation apparatus.
